# EUROPÄISCHE PATENTSCHRIFT

(11) **EP 1 222 029 B1**
(45) Veröffentlichungstag und Bekanntmachung des Hinweises auf die Patenterteilung: **26.05.2004**
(21) Anmeldenummer: 00978971.0
(22) Anmeldetag: 04.10.2000
(51) Int. Cl.: B01L 3/00, G02B 1/10

(54) **SUBSTRAT MIT MINDESTENS EINER PORE**
SUBSTRATE WITH AT LEAST ONE PORE
SUBSTRAT COMPORTANT AU MOINS UN PORE

(30) Priorität: 06.10.1999 DE 19948089
(43) Veröffentlichungstag der Anmeldung: 17.07.2002
(73) Patentinhaber: Infineon Technologies AG, 81669 München (DE)
(72) Erfinder: LEHMANN, Volker, 80689 München (DE)
(74) Vertreter: Viering, Jentschura & Partner
(86) Internationale Anmeldenummer: PCT/DE2000/003490
(87) Internationale Veröffentlichungsnummer: WO 2001/024929

(56) Entgegenhaltungen:
- EP-A- 0 391 578
- EP-A- 0 980 066
- WO-A-99/42608
- US-A- 4 919 778
- US-A- 5 843 767
- US-A- 5 914 804
- "handbook of optics, volume 1, fundamentals, techniques and design, Ed 2" 1995 , MCGRAW-HILL, INC , US, NEW YORK XP002161512 Seite 42.14, Absatz 3 - Absatz 4

## Beschreibung

Mit einem Biochip kann eine Lösung aus DNA-Sequenzen auf Vorhandensein bestimmter DNA-Sequenzen untersucht werden. Dazu werden zu jeder nachzuweisenden DNA-Sequenz dazu komplementäre DNA-Sequenzen erzeugt, auf ein Gebiet eines Substrats des Biochips aufgebracht und mittels einer Haftschicht unbeweglich gemacht. Jeder nachzuweisenden DNA-Sequenz ist ein verschiedenes Gebiet des Substrats zugeordnet. Jede DNA-Sequenz der Lösung wird durch ein chemisches Verfahren mit einem fluoreszierenden Molekül verbunden. Anschließend wird die Lösung auf das Substrat des Biochips aufgebracht. Aus der Lösung der DNA-Sequenzen binden nur die nachzuweisenden DNA-Sequenzen an die jeweils komplementären DNA-Sequenzen. Nach Entfernung der übrigbleibenden Lösung wird Licht auf das Substrat eingestrahlt und gemessen, ob und aus welchen Gebieten des Substrats Licht von den fluoreszierenden Moleküle emittiert wird. Da jedem Gebiet einer bestimmten nachzuweisenden DNA-Sequenz zugeordnet ist, kann nicht nur bestimmt werden, ob in der Lösung nachzuweisende DNA-Sequenzen vorhanden sind, sondern auch welche der nachzuweisenden DNA-Sequenzen in der Lösung vorhanden sind.

Von der Firma Affimetrix werden Biochips vertrieben, die Substrate mit planaren Oberflächen aufweisen.

Zur Erhöhung der Nachweisempfindlichkeit der nachzuweisenden DNA-Sequenzen und damit der fluoreszierenden Moleküle wird in US 5 843 767 A vorgeschlagen, als Substrat eines Biochips ein poröses Glas- oder Siliziumsubstrat zu verwenden. Durch die Poren wird die effektive Oberfläche des Substrats vergrößert, so daß größere Gebiete des Substrats mit den komplementären DNA-Sequenzen versehen werden können, die folglich mehr nachzuweisende DNA-Sequenzen binden können, wodurch die Menge an durch Fluoreszenz emittiertem Licht pro Gebiet, das heißt pro nachzuweisender DNA-Sequenz erhöht wird. Damit die Lösung besonders leicht mit der gesamten effektiven Oberfläche des Substrats in Kontakt kommt, reichen die Poren von einer Oberfläche des Substrats bis zu einer der Oberfläche gegenüberliegenden Oberfläche des Substrats. Die Lösung kann durch die Poren hindurchfließen und kommt so mit Oberflächen der Poren in Kontakt.

Der Erfindung liegt das Problem zugrunde, ein Substrat mit mindestens einer Pore anzugeben, das als Teil eines Biochips mit im Vergleich zum Stand der Technik erhöhter Nachweisempfindlichkeit fluoreszierender Moleküle geeignet ist.

Das Problem wird gelöst durch ein Substrat mit mindestens einer Pore, bei dem die Pore eine innere Oberfläche aufweist, welche mit einer Beschichtung zur Reflexion elektromagnetischer Strahlung versehen ist, so dass elektromagnetische Strahlung von einem Ende der Pore bis zum anderen Ende der Pore mittels Reflexion geleitet werden kann. Damit eine Lösung von fluoreszierenden Molekülen besonders einfach an die gesamte Oberfläche der Pore gelangt, erstreckt sich die Pore von einer Oberfläche des Substrats bis zu einer der Oberfläche gegenüberliegenden Oberfläche des Substrats. Die Lösung kann somit durch die Pore hindurch gepumpt werden.

Das Substrat kann weitere Poren aufweisen, die wie die Pore ausgestaltet sind. Das Substrat kann Teil eines Biochips zum Nachweis von auf die mit der Beschichtung versehenen Oberflächen der Poren aufgebrachten fluoreszierenden Molekülen sein.

Die durch Fluoreszenz der fluoreszierenden Moleküle emittierte elektromagnetische Strahlung, die beim Nachweis der fluoreszierenden Moleküle gemessen wird, geht aufgrund der Beschichtung bis zum Austritt aus den Poren weniger stark verloren. Die emittierte Strahlung wird aufgrund der Beschichtung innerhalb der Poren stärker reflektiert und weniger absorbiert, so daß die Menge an gemessener elektromagnetischer Strahlung erhöht wird, und folglich die Nachweisempfindlichkeit erhöht wird.

Das Substrat kann auch in anderer Weise als zum Nachweis fluoreszierender Moleküle verwendet werden. Beispielsweise ist das Substrat ein Lichtleiter oder Teil eines Lichtleiters, der Licht vom einen Ende der Pore bis zum anderen Ende der Pore durch Reflexion leitet. Gegenüber einer Glasfaser weist ein solcher Lichtleiter den Vorteil auf, daß Licht beim Eintritt und beim Austritt keine Grenzfläche zwischen einem Material des Lichtleiters und Luft durchqueren muß, die in der Regel reflektiert und folglich die Lichtintensität erniedrigt.

Ist das Substrat Teil eines Biochips, bei dem fluoreszierende Moleküle nachgewiesen werden sollen, die elektromagnetische Strahlung mit Wellenlängen zwischen 700 nm und 400 nm emittieren, ist die Beschichtung vorzugsweise derart ausgestaltet, daß sie elektromagnetische Strahlung eben dieser Wellenlängen optimal reflektiert.

Je öfter die emittierte elektromagnetische Strahlung innerhalb der Pore bis zum Verlassen der Pore reflektiert wird, umso kleiner ist deren Intensität. Da die Reflektivität der Beschichtung auch vom Einfallswinkel der elektromagnetischen Strahlung abhängt, kann die Beschichtung für bestimmte Einfallswinkel optimiert werden. Vorzugsweise wird die Beschichtung für Einfallswinkel optimiert, bei denen die elektromagnetische Strahlung im Mittel nur etwa drei oder vier Mal reflektiert wird. Die Optimierung hängt dabei auch von Abmessungen der Pore, wie deren Tiefe und deren Durchmesser, ab.

Bei der Optimierung können auch die Apertur der zum Nachweis der emittierten Strahlung sammelnden Optik sowie deren Entfernung vom Substrat berücksichtigt werden. Zur sammelnden Optik, die optische Linsen umfaßt, kann nur Strahlung auftreffen, die unter einem Winkel emittiert wurde, der größer als ein von der Entfernung abhängiger Mindestwinkel ist. Die Optimierung geschieht vorzugsweise für solche Winkel.

Darüber hinaus können bei der Optimierung auch der Brechungsindex des in der Pore befindlichen Mediums und eventuell vorhandene Zwischenschichten zwischen dem Substrat und der sammelnden Optik berücksichtigt werden. Auch die Wellenlänge der anregenden elektromagnetischen Strahlung kann berücksichtigt werden.

Vorzugsweise ist die Beschichtung derart ausgestaltet, daß sie elektromagnetische Strahlung, die unter einem Einfallswinkel von zwischen 50° und 90° auf die Beschichtung einfällt, optimal reflektiert. Insbesondere kann dieser Einfallswinkel etwa 70° betragen.

Das Substrat besteht beispielsweise aus Glas.

Vorzugsweise besteht das Substrat aus Silizium. Silizium ist im Vergleich zu Glas einfacher zu strukturieren. Darüber hinaus vermischt sich elektromagnetische Strahlung verschiedener Poren, die zu Gebieten des Substrats gehören, die mit verschiedenen komplementären DNA-Sequenzen versehen sind, im Vergleich zu Glas kaum, da Silizium für elektromagnetische Strahlung eines großen Frequenzbereichs undurchlässig ist.

Besteht das Substrat aus Silizium, so ist die Beschichtung vorzugsweise derart ausgestaltet, daß sie mit Standardprozessen der Mikroelektronik herstellbar ist.

Die Beschichtung kann beispielsweise aus einer einzelnen Schicht bestehen.

Die Beschichtung weist eine besonders gute Reflektivität auf, wenn sie Metall enthält. Vorzugsweise enthält die Beschichtung Wolfram, da Wolfram mit Hilfe eines CVD-Prozesses auf Oberflächen auch von Poren, die tiefer als 10 µm sind und zugleich einen weniger als ca. 1000 nm großen Durchmesser aufweisen, aufgebracht werden kann.

Als Beschichtung ist auch eine dielektrische Schicht geeignet, deren elektrische Dielektrizitätskonstante größer ist als die des Substrats.

Zur Erhöhung der Reflektivität kann die Beschichtung aus mindestens zwei übereinander angeordneten Schichten bestehen. Vorzugsweise weisen die Schichten abwechselnd eine hohe Dielektrizitätskonstante und eine niedrige Dielektrizitätskonstante auf.

Vorzugsweise besteht mindestens eine der Schichten aus SiO₂, Polysilizium oder Siliziumnitrid, da diese Materialien mit Hilfe von Standardprozessen der Mikroelektronik erzeugt werden können.

vorzugsweise besteht die äußerste Schicht der Beschichtung, das heißt jene Schicht, in der die elektromagnetische Strahlung zuerst einfällt, aus SiO₂ oder aus Polysilizium, da Methoden bekannt sind, mit denen die komplementären DNA-Sequenzen auf diesen Materialien befestigt werden können.

Vorzugsweise bestehen alle Schichten der Beschichtung aus SiO₂, Polysilizium oder Siliziumnitrid.

Eine hohe Reflektivität ist bei zugleich kleinem Prozeßaufwand erzielbar, wenn die Beschichtung aus einer ersten Schicht, die auf dem Substrat angeordnet ist, und einer auf der ersten Schicht angeordneten zweiten Schicht besteht.

Vorzugsweise besteht die erste Schicht aus SiO₂ oder aus Siliziumnitrid. Die zweite Schicht besteht vorzugsweise aus Polysilizium.

Eine bessere Reflektivität wird erzielt, wenn die Beschichtung aus einer ersten Schicht, die auf dem Substrat angeordnet ist, einer auf der ersten Schicht angeordneten zweiten Schicht und einer auf der zweiten Schicht angeordneten dritten Schicht besteht.

Beispielsweise bestehen die erste Schicht und die dritte Schicht aus Siliziumnitrid oder aus SiO₂. Die zweite Schicht besteht beispielsweise aus Polysilizium.

Eine noch höhere Reflektivität wird erzielt, wenn die Beschichtung aus einer ersten Schicht, die auf dem Substrat angeordnet ist, einer auf der ersten Schicht angeordneten zweiten Schicht, einer auf der zweiten Schicht angeordneten dritten Schicht und einer auf der dritten Schicht angeordneten vierten Schicht besteht.

Die erste Schicht und die dritte Schicht bestehen beispielsweise aus Siliziumnitrid oder aus SiO₂. Die zweite Schicht und die vierte Schicht bestehen beispielsweise aus Polysilizium.

Bei der Optimierung der Beschichtung werden die Dicken ihrer Schichten an die jeweiligen Erfordernisse angepaßt.

Im folgenden werden Ausführungsbeispiele der Erfindung anhand der Figuren näher erläutert.
- Figur 1: zeigt einen Querschnitt durch ein erstes Substrat mit Poren, einer ersten Schicht und einer zweiten Schicht. Ferner werden der Verlauf von Licht, das ein fluoreszierendes Molekül anregt, und der Verlauf von vom fluoreszierenden Molekül emittiertem Licht dargestellt.
- Figur 2a: zeigt die Abhängigkeit der Reflektivität des beschichteten ersten Substrats in Abhängigkeit vom Einfallswinkel und von der Wellenlänge der zu reflektierenden elektromagnetischen Strahlung in einer dreidimensionalen Darstellung.
- Figur 2b: zeigt die Abhängigkeit aus Figur 2a in einer zweidimensionalen Darstellung.
- Figur 3a: zeigt die Abhängigkeit der Reflektivität einer unbeschichteten Siliziumoberfläche vom Einfallswinkel und von der Wellenlänge der zu reflektierenden elektromagnetischen Strahlung in einer dreidimensionalen Darstellung.
- Figur 3b: zeigt die Abhängigkeit aus Figur 3a in einer zweidimensionalen Darstellung.
- Figur 4: zeigt einen Querschnitt durch ein zweites Substrat mit einer ersten Schicht, einer zweiten Schicht und einer dritten Schicht.
- Figur 5a: zeigt die Reflektivität des beschichteten zweiten Substrats in Abhängigkeit vom Einfallswinkel und von der Wellenlänge der zu reflektierenden elektromagnetischen Strahlung in einer dreidimensionalen Darstellung.
- Figur 5b: zeigt die Abhängigkeit aus Figur 5a in einer zweidimensionalen Darstellung.
- Figur 6: zeigt einen Querschnitt durch ein drittes Substrat mit einer ersten Schicht, einer zweiten Schicht, einer dritten Schicht und einer vierten Schicht.
- Figur 7a: zeigt die Abhängigkeit der Reflektivität eines beschichteten dritten Substrats in Abhängigkeit vom Einfallswinkel und von der Wellenlänge der zu reflektierenden Strahlung in einer dreidimensionalen Darstellung.
- Figur 7b: zeigt die Abhängigkeit aus Figur 7a in einer zweidimensionalen Darstellung.

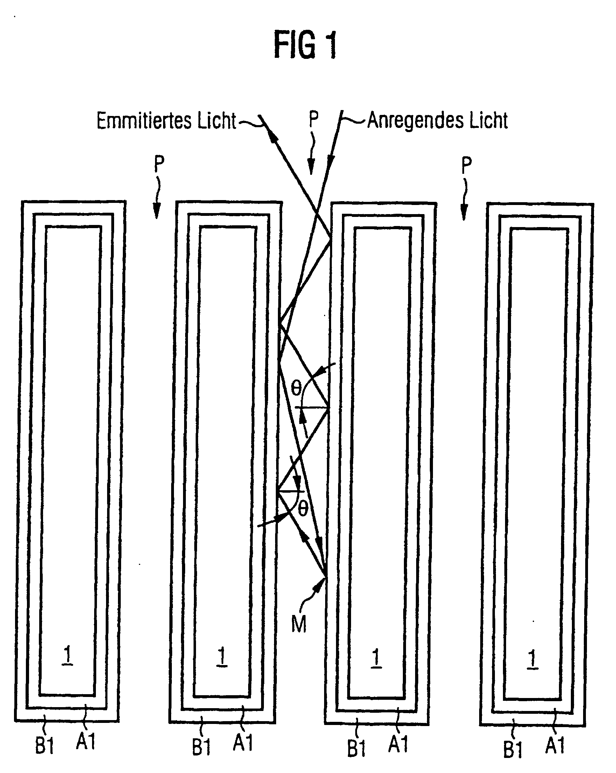

- Figuren 1, 4 und 6: sind nicht maßstabsgetreu.

In einem ersten Ausführungsbeispiel ist als Teil eines ersten Biochips ein erstes Substrat 1 aus Silizium vorgesehen, in dem Poren P angeordnet sind, die von einer Oberfläche des ersten Substrats 1 bis zu einer der Oberfläche gegenüberliegenden Oberfläche des ersten Substrats 1 reichen (siehe Figur 1). Die Poren P sind ca. 500 µm tief und weisen einen Durchmesser von ca. 10µm auf.

Oberflächen der Poren und des Substrats sind mit einer Beschichtung zur Reflexion elektromagnetischer Strahlung versehen, die aus einer ersten Schicht A1 und einer darauf angeordneten zweiten Schicht B1 besteht (siehe Figur 1). Die erste Schicht A1 ist ca. 150 nm dick und besteht aus SiO₂. Durch thermische Oxidation wird die erste Schicht A1 aus SiO₂ erzeugt. Die zweite Schicht B1 ist ca. 29 nm dick und besteht aus Polysilizium. Die zweite Schicht B1 wird durch Abscheiden von Polysilizium in einem CVD-Prozeß erzeugt.

Figur 1 zeigt, wie mit dem Biochip fluoreszierende Moleküle nachgewiesen werden können. Anregendes Licht mit einer Wellenlänge von ca. 400 nm fällt in eine der Poren P ein, auf deren mit der Beschichtung versehene Oberfläche ein fluoreszierendes Molekül M angeordnet ist. Das fluoreszierende Molekül M wird durch das anregende Licht angeregt und emittiert Licht mit einer Wellenlänge von ca. 600 nm mit einer bestimmten Wahrscheinlichkeit in einer solchen Richtung, daß das emittierte Licht unter einem Einfallswinkel von Θ = 70° auf die Oberfläche der Pore P auftrifft und viermal reflektiert wird, bis das emittierte Licht die Pore P verläßt und nachgewiesen werden kann. Die Dicken der Schichten A1, B1 sind derart gewählt, daß für elektromagnetische Strahlung, die unter einem Winkel von ca. 70° einfällt und eine Wellenlänge zwischen ca. 450 nm bis 660 nm aufweist, eine optimale Reflektivität erzielt wird.

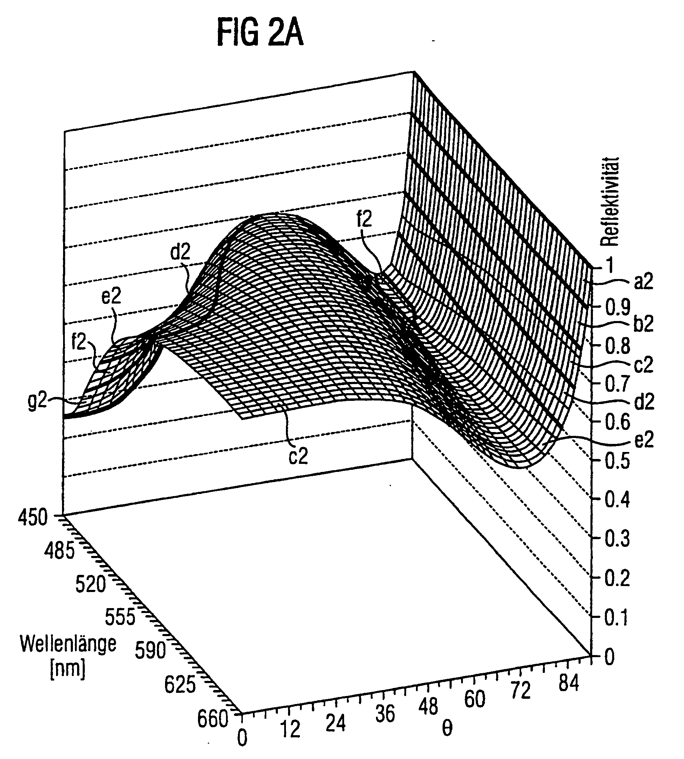

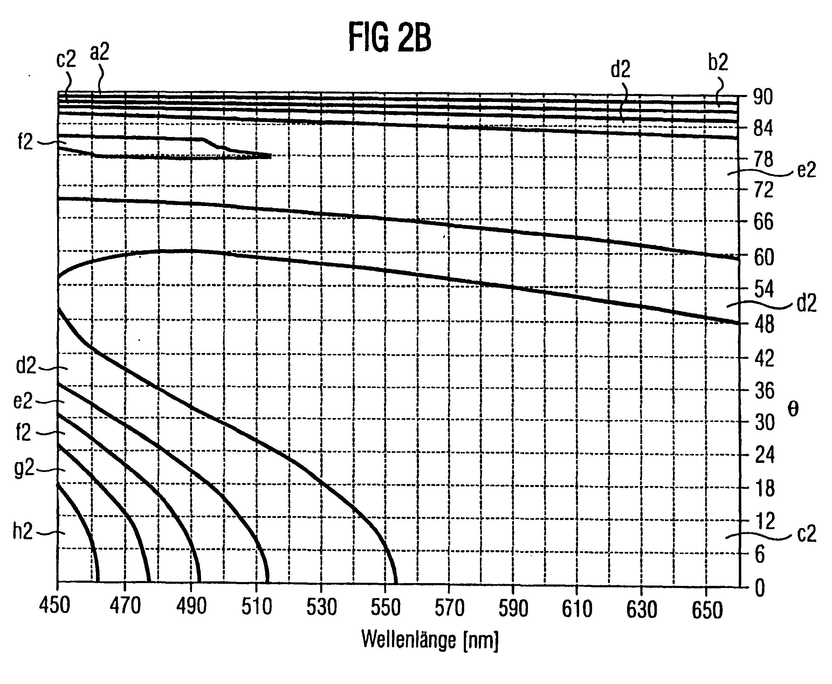

Figuren 2a und 2b zeigen die Reflektivität des beschichteten ersten Substrats 1 in Abhängigkeit vom Einfallswinkel und von der Wellenlänge der zu reflektierenden elektromagnetischen Strahlung. Die Reflektivität ist das Verhältnis der Intensität der elektromagnetischen Strahlung nach der Reflexion zur Intensität der elektromagnetischen Strahlung vor der Reflexion. Flächen, die mit a2, bezeichnet sind, stellen Reflektivitäten zwischen 0,9 und 1 dar (a2 = 0,9 bis 1). Analog gilt:
b2 = 0,8 bis 0,9
c2 = 0,7 bis 0,8
d2 = 0,6 bis 0,7
e2 = 0,5 bis 0,6
f2 = 0,4 bis 0,5
g2 = 0,3 bis 0,4
h2 = 0,2 bis 0,3

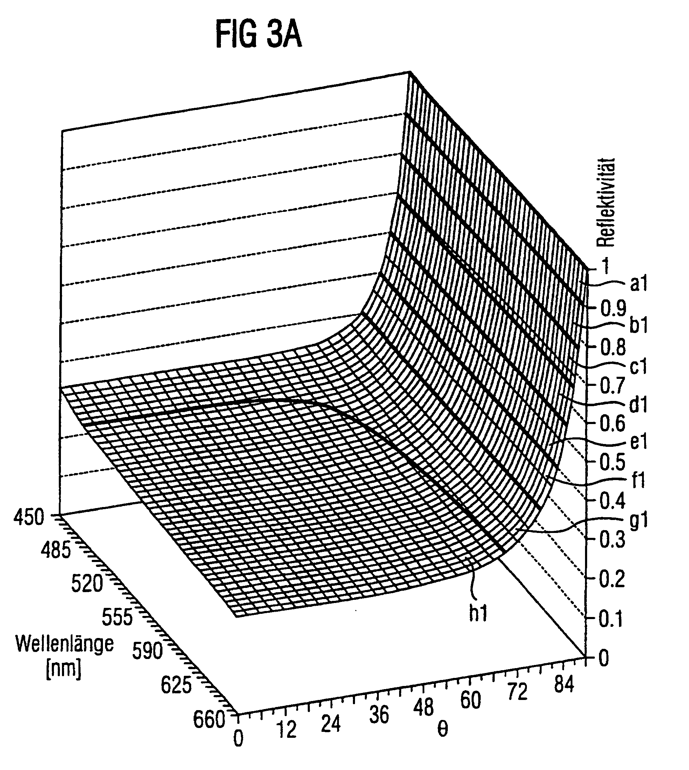

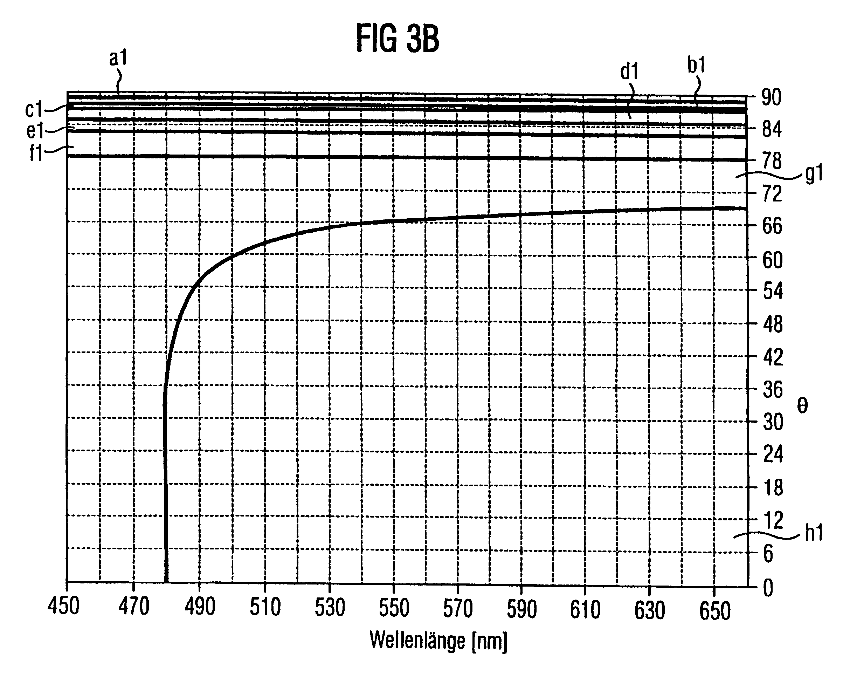

Figuren 3a und 3b zeigen die Abhängigkeit der Reflektivität einer Siliziumoberfläche ohne Beschichtung in Abhängigkeit vom Einfallswinkel und von der Wellenlänge der zu reflektierenden elektromagnetischen Strahlung. Für die Bezeichnung der Flächen gilt:
a1 = 0,9 bis 1
b1 = 0,8 bis 0,9
c1 = 0,7 bis 0,8
d1 = 0,6 bis 0,7
e1 = 0,5 bis 0,6
f1 = 0,4 bis 0,5
g1 = 0,3 bis 0,4
h1 = 0,2 bis 0,3

Ein Vergleich der Figuren 2a und 2b mit den Figuren 3a und 3b zeigt, daß die Reflektivität des ersten Substrats 1 für fast alle Winkel, das heißt für Winkel kleiner als ca. 85°, und für Wellenlängen zwischen 450 nm und 660 nm gegenüber der unbeschichteten Siliziumoberfläche stark erhöht ist.

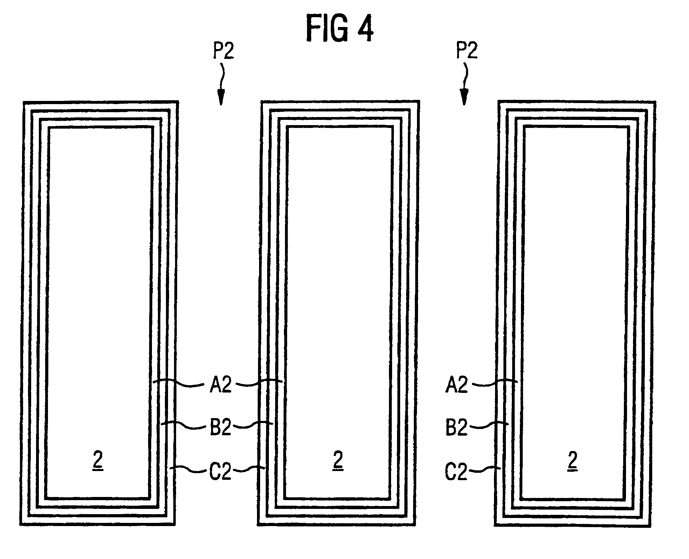

In einem zweiten Ausführungsbeispiel ist ein zweiter Biochip mit einem zweiten Substrat 2 vorgesehen, der wie der erste Biochip ausgestaltet ist mit dem Unterschied, daß die Beschichtung aus einer ersten Schicht A2, einer darauf angeordneten zweiten Schicht B2 und einer darauf angeordneten dritten Schicht C2 besteht (siehe Figur 4). Die erste Schicht A2 ist ca. 185 nm dick und besteht aus SiO₂. Die zweite Schicht B2 ist ca. 33 nm dick und besteht aus Polysilizium. Die dritte Schicht C2 ist ca. 134 nm dick und besteht aus Siliziumnitrid. Die Dicken der Schichten A2, B2, C2 sind derart gewählt, daß für elektromagnetische Strahlung, die unter einem Winkel von ca. 70° einfällt und eine Wellenlänge zwischen ca. 450nm bis 660nm aufweist, eine optimale Reflektivität erzielt wird.

Da sich die Wellenlängen auf Luft beziehen, können sich bei der Optimierung für die Dicken der Schichten A2, B2, C2 etwas veränderte Werte ergeben, wenn man von einem anderen Medium als Luft, zum Beispiel einer wasserhaltigen Lösung, in den Poren P2 ausgeht. Das Entsprechende gilt auch für die anderen Ausführungsbeispiele.

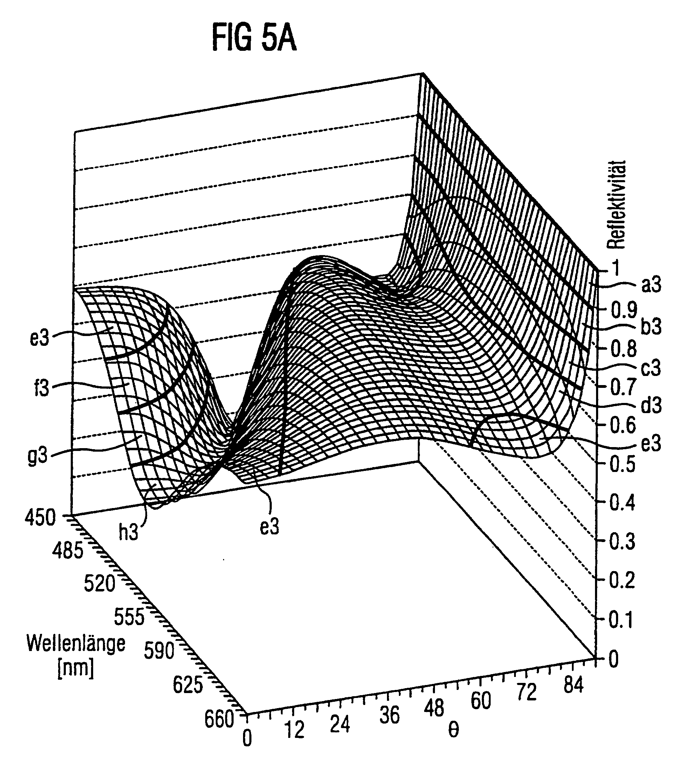

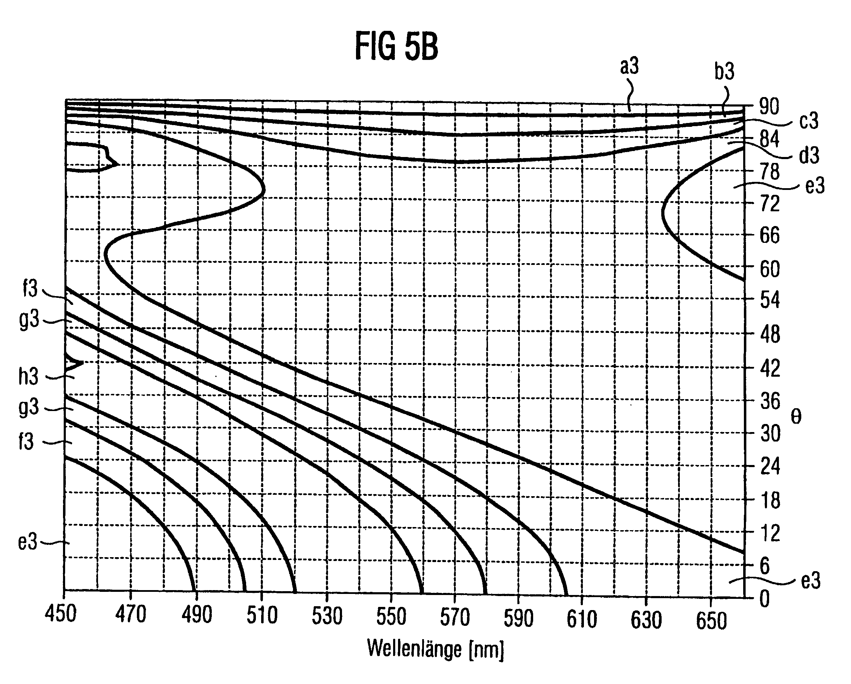

Ein Vergleich der Figuren 2a und 2b mit den Figuren 5a und 5b zeigt, daß die Reflektivität des zweiten Substrats 2 gegenüber der Reflektivität des ersten Substrats 1 für einige Wellenlängen und Einfallswinkel verbessert ist. Für die Bezeichnung der Flächen in den Figuren 5a und 5b gilt:
a3 = 0,9 bis 1
b3 = 0,8 bis 0,9
c3 = 0,7 bis 0,8
d3 = 0,6 bis 0,7
e3 = 0,5 bis 0,6
f3 = 0,4 bis 0,5
g3 = 0,3 bis 0,4
h3 = 0,2 bis 0,3

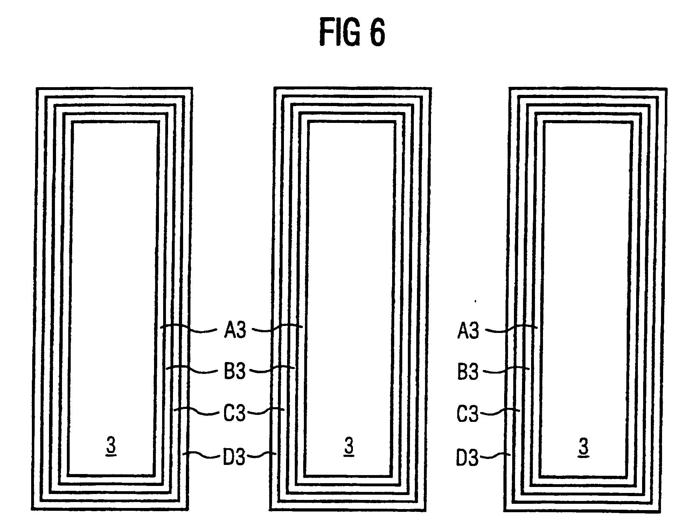

In einem dritten Ausführungsbeispiel ist ein dritter Biochip mit einem dritten Substrat 3 vorgesehen, der analog zum ersten Biochip ausgestaltet ist mit dem Unterschied, daß die Beschichtung aus einer ersten Schicht A3, einer darauf angeordneten zweiten Schicht B3, einer darauf angeordneten dritten Schicht C3 und einer darauf angeordneten vierten Schicht D3 besteht (siehe Figur 6). Die erste Schicht A3 ist ca. 191 nm dick und besteht aus SiO₂. Die zweite Schicht B3 ist ca. 33 nm dick und besteht aus Polysilizium. Die dritte Schicht C3 ist ca. 93 nm dick und besteht aus Siliziumnitrid. Die vierte Schicht D3 ist ca. 27 nm dick und besteht aus Polysilizium. Die Dicken der Schichten A3, B3, C3, D3 sind derart gewählt, daß elektromagnetische Strahlung, die unter einem Einfallswinkel von 70 ° einfällt und eine Wellenlänge zwischen 450 nm und 660 nm aufweist, optimal reflektiert wird.

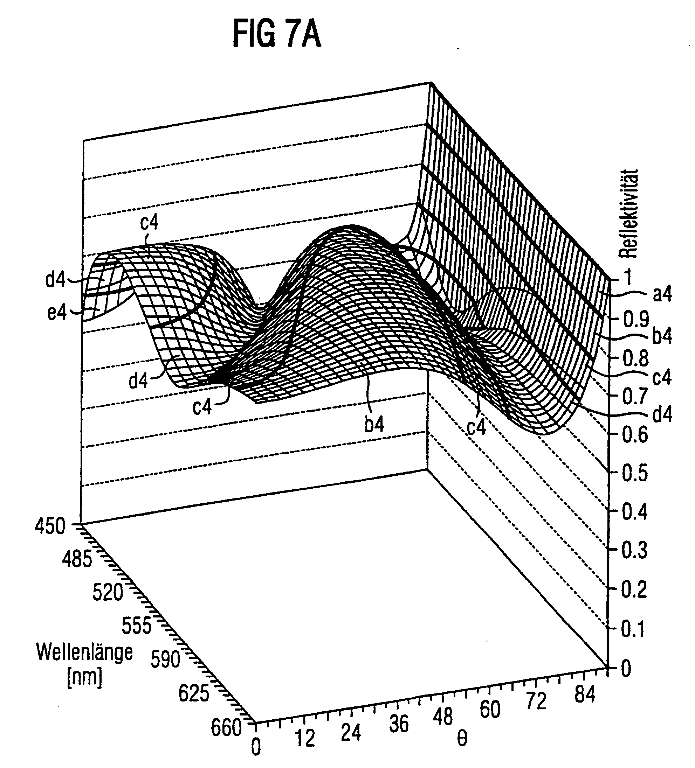

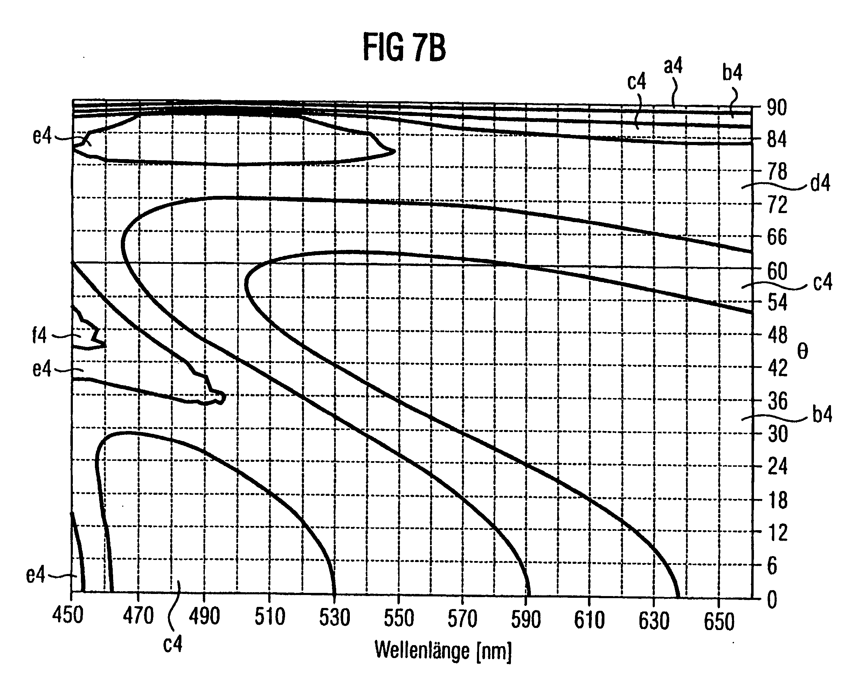

Ein Vergleich der Figuren 5a und 5b mit den Figuren 7a und 7b zeigt, daß die Reflektivität des dritten Substrats 3 gegenüber der Reflektivität des zweiten Substrats 2 erhöht ist. Für die Bezeichnung der Flächen in den Figuren 7a und 7b gilt:
a4 = 0,9 bis 1
b4 = 0,8 bis 0,9
c4 = 0,7 bis 0,8
d4 = 0,6 bis 0,7
e4 = 0,5 bis 0,6
f4 = 0,4 bis 0,5

Im Rahmen der Erfindung kann als Variation der Ausführungsbeispiele die Beschichtung aus mehr als vier Schichten bestehen. Für die Schichten der drei Ausführungsbeispiele können andere Materialien gewählt werden. Die Dicken der Schichten der drei Ausführungsbeispiele können für elektromagnetische Strahlung mit anderen Einfallswinkeln und anderen Wellenlängen optimiert werden. Poren der drei Ausführungsbeispiele sind auch als Lichtleiter geeignet. In diesem Fall können die Poren gekrümmt sein.

## Patentansprüche

1. Substrat mit mindestens einer Pore, bei dem sich die Pore (P; P2) von einer Oberfläche des Substrats (1; 2; 3) bis zu einer der Oberfläche gegenüberliegenden Oberfläche des Substrats (1; 2; 3) erstreckt, so dass eine Lösung durch die Pore (P; P2) hindurch gepumpt werden kann,
**dadurch gekennzeichnet, dass**
die Pore (P; P2) eine innere Oberfläche aufweist, welche mit einer Beschichtung zur Reflexion elektromagnetischer Strahlung versehen ist, so dass elektromagnetische Strahlung von einem Ende der Pore (P; P2) bis zum anderen Ende der Pore (P; P2) mittels Reflexion geleitet werden kann.

2. Substrat nach Anspruch 1, bei dem die Beschichtung Wolfram enthält.

3. Substrat nach Anspruch 1 oder 2, bei dem die Beschichtung aus mindestens zwei übereinander angeordneten Schichten (A1, B1; A2, B2, C3; A3, B3, C3, D3) besteht.

4. Substrat nach Anspruch 3, bei dem mindestens eine der Schichten (A1, B1; A2, B2, C2; A3, B3, C3, D3) aus SiO₂, Polysilizium oder Siliziumnitrid besteht.

5. Substrat nach Anspruch 4, bei dem die Beschichtung aus einer ersten Schicht (A1), die auf dem Substrat (1) angeordnet ist, und einer auf der ersten Schicht (A1) angeordneten zweiten Schicht (B1) besteht.

6. Substrat nach Anspruch 5, bei dem die erste Schicht (A1) aus SiO₂ oder aus Siliziumnitrid besteht, und bei dem die zweite Schicht (B1) aus Polysilizium besteht.

7. Substrat nach Anspruch 4, bei dem die Beschichtung aus einer ersten Schicht (A2), die auf dem Substrat (2) angeordnet ist, einer auf der ersten Schicht (A2) angeordneten zweiten Schicht (B2) und einer auf der zweiten Schicht (B2) angeordneten dritten Schicht (C2) besteht.

8. Substrat nach Anspruch 7, bei dem die erste Schicht (A2) und die dritte Schicht (C2) aus Siliziumnitrid oder aus SiO₂ bestehen, und bei dem die zweite Schicht (B2) aus Polysilizium besteht.

9. Substrat nach Anspruch 4, bei dem die Beschichtung aus einer ersten Schicht (A3), die auf dem Substrat (3) angeordnet ist, einer auf der ersten Schicht (A3) angeordneten zweiten Schicht (B3), einer auf der zweiten Schicht (B3) angeordneten dritten Schicht (C3) und einer auf der dritten Schicht (C3) angeordneten vierten Schicht (D3) besteht.

10. Substrat nach Anspruch 9, bei dem die zweite Schicht (B3) und die vierte Schicht (D3) aus Polysilizium bestehen, und bei dem die erste Schicht (A3) und die dritte Schicht (C3) aus Siliziumnitrid oder aus SiO₂ bestehen.

11. Substrat nach einem der Ansprüche 1 bis 10, mit weiteren Poren (P; P2), die wie die Pore (P; P2) ausgestaltet sind, wobei das Substrat das Teil eines Biochips zum Nachweis von auf die mit der Beschichtung versehenen Oberflächen der Poren (P; P2) aufgebrachten fluoreszierenden Molekülen (M) ist.

12. Substrat nach einem der Ansprüche 1 bis 10, das zumindest Teil eines Lichtleiters ist.

13. Substrat nach einem der Ansprüche 1 bis 12, bei dem die Beschichtung derart ausgestaltet ist, daß sie elektromagnetische Strahlung mit Wellenlängen zwischen 400 nm und 700 nm im Wesentlichen reflektiert.

14. Substrat nach einem der Ansprüche 1 bis 13, bei dem das Substrat (1; 2; 3) im Wesentlichen aus Silizium besteht.

## Claims

1. A substrate with at least one pore,
- in which a surface of the pore (P; P2) extends from one surface of the substrate (1; 2; 3) as far as a surface of the substrate (1; 2; 3) lying opposite the surface, so that a solution can be pumped through the pore (P; P2),
**characterized in that**
the pore (P; P2) has an inner surface which is provided with a coating for reflecting electromagnetic radiation, so that electromagnetic radiation can be conducted from one end of the pore (P; P2) to the other end of the pore (P; P2) by reflection.

2. The substrate as claimed in claim 1,
- in which the coating contains tungsten.

3. The substrate as claimed in claim 1 or 2,
- in which the coating consists of at least two layers (A1, B1; A2, B2, C3; A3, B3, C3, D3) arranged above one another.

4. The substrate as claimed in claim 3,
- in which at least one of the layers (A1, B1; A2, B2, C2; A3, B3, C3, D3) consists of SiO₂, polysilicon or silicon nitride.

5. The substrate as claimed in claim 4,
- in which the coating consists of a first layer (A1), which is arranged on the substrate (1), and a second layer (B1) arranged on the first layer (A1).

6. The substrate as claimed in claim 5,
- in which the first layer (A1) consists of SiO₂ or silicon nitride, and
- in which the second layer (B1) consists of polysilicon.

7. The substrate as claimed in claim 4,
- in which the coating consists of a first layer (A2), which is arranged on the substrate (2), a second layer (B2) arranged on the first layer (A2), and a third layer (C2) arranged on the second layer (B2).

8. The substrate as claimed in claim 7,
- in which the first layer (A2) and the third layer (C2) consist of silicon nitride or SiO₂, and
- in which the second layer (B2) consists of polysilicon.

9. The substrate as claimed in claim 4,
- in which the coating consists of a first layer (A3), which is arranged on the substrate (3), a second layer (B3) arranged on the first layer (A3), a third layer (C3) arranged on the second layer (B3), and a fourth layer (D3) arranged on the third layer (C3).

10. The substrate as claimed in claim 9,
- in which the second layer (B3) and the fourth layer (D3) consist of polysilicon, and
- in which the first layer (A3) and the third layer (C3) consist of silicon nitride or SiO₂.

11. The substrate as claimed in one of claims 1 to 10,
- with further pores (P; P2), which are configured like the pore (P; P2), in which the substrate is part of a biochip for detecting fluorescent molecules (M) applied to the surfaces of the pores (P; P2) provided with the coating.

12. The substrate as claimed in one of claims 1 to 10,
- which is at least part of an optical waveguide.

13. The substrate as claimed in one of claims 1 to 12,
- in which the coating is configured in such a way that it essentially reflects electromagnetic radiation with wavelengths between 400 nm and 700 nm.

14. The substrate as claimed in one of claims 1 to 13,
- in which the substrate (1; 2; 3) essentially consists of silicon.

## Revendications

1. Substrat comportant au moins un pore, où le pore (P ; P2) s'étend d'une surface du substrat (1 ; 2 ; 3) jusqu'à une surface opposée à la surface du substrat (1 ; 2 ; 3), de sorte qu'une solution peut être pompée par le pore (P ; P2), **caractérisé en ce que** le pore (P ; P2) présente une surface interne, qui est munie d'un revêtement pour la réflexion d'un rayonnement électromagnétique, de sorte que le rayonnement électromagnétique peut être conduit d'une extrémité du pore (P ; P2) jusqu'à l'autre extrémité du pore (P ; P2) par réflexion.

2. Substrat selon la revendication 1, dans lequel le revêtement contient du tungstène.

3. Substrat selon la revendication 1 ou 2, dans lequel le revêtement consiste en au moins deux couches superposées (A1, B1 ; A2, B2, C2 ; A3, B3, C3, D3).

4. Substrat selon la revendication 3, dans lequel au moins une des couches (A1, B1 ; A2, B2, C2 ; A3, B3, C3, D3) consiste en du SiO₂, du polysilicium ou du nitrure de silicium.

5. Substrat selon la revendication 4, dans lequel le revêtement consiste en une première couche (A1), qui est disposée sur le substrat (1), et en une deuxième couche (B1) disposée sur la première couche (A1).

6. Substrat selon la revendication 5, dans lequel la première couche (A1) consiste en du SiO₂ ou du nitrure de silicium, et dans lequel la deuxième couche (B1) consiste en du polysilicium.

7. Substrat selon la revendication 4, dans lequel le revêtement consiste en une première couche (A2), qui est disposée sur le substrat (2), en une deuxième couche (B2) disposée sur la première couche (A2) et en une troisième couche (C2) disposée sur la deuxième couche (B2).

8. Substrat selon la revendication 7, dans lequel la première couche (A2) et la troisième couche (C2) consistent en du nitrure de silicium ou du SiO₂, et dans lequel la deuxième couche (B2) consiste en du polysilicium.

9. Substrat selon la revendication 4, dans lequel le revêtement consiste en une première couche (A3), qui est disposée sur le substrat (3), en une deuxième couche (B3) disposée sur la première couche (A3), en une troisième couche (C3) disposée sur la deuxième couche (B3) et en une quatrième couche (D3) disposée sur la troisième couche (C3).

10. Substrat selon la revendication 9, dans lequel la deuxième couche (B3) et la quatrième couche (D3) consistent en du polysilicium, et dans lequel la première couche (A3) et la troisième couche (C3) consistent en du nitrure de silicium ou du SiO₂.

11. Substrat selon l'une quelconque des revendications 1 à 10, avec d'autres pores (P ; P2), qui sont équipés comme le pore (P ;P2), où le substrat est une partie d'une biopuce pour la détection de molécules fluorescentes (M) appliquées sur les surfaces des pores (P ; P2), munies du revêtement.

12. Substrat selon l'une quelconque des revendications 1 à 10, qui est au moins une partie d'un conducteur de la lumière.

13. Substrat selon l'une quelconque des revendications 1 à 12, dans lequel le revêtement est aménagé de sorte qu'il réfléchit essentiellement, un rayonnement électromagnétique avec des longueurs d'onde situées dans l'intervalle allant de 400 nm à 700 nm.

14. Substrat selon l'une quelconque des revendications 1 à 13, dans lequel le substrat (1 ; 2 ; 3) consiste essentiellement en du silicium.
